# EUROPEAN PATENT APPLICATION

(11) **EP 4 671 354 A1**
(43) Date of publication of application: **31.12.2025**
(21) Application number: 24810960.5
(22) Date of filing: 13.05.2024
(51) Int. Cl.: C12M 1/36

(54) **CULTURE DEVICE AND METHOD FOR CALIBRATING HUMIDITY SENSOR**

(30) Priority: 24.05.2023 JP 2023085709
(71) Applicant: PHC HOLDINGS CORPORATION, Tokyo 100-8403 (JP)
(72) Inventor: TEMMAN, Haruka, Toon-shi, Ehime 791-0395 (JP); OHTA, Akihiro, Toon-shi, Ehime 791-0395 (JP); HITOMI, Tsugumasa, Toon-shi, Ehime 791-0395 (JP); SAKAI, Yuta, Toon-shi, Ehime 791-0395 (JP)
(74) Representative: Grünecker Patent- und Rechtsanwälte PartG mbB
(86) International application number: PCT/JP2024/017703
(87) International publication number: WO 2024/241953

(57) **Abstract**

A culture apparatus includes: a first vapor supplier that humidifies a culture chamber; a humidity sensor that detects a humidity in the culture chamber; and a calibrator that brings the humidity in the culture chamber to a known equilibrium humidity by the first vapor supplier and calibrates the humidity sensor based on a measured value of the humidity sensor and the equilibrium humidity.

## Description

### Technical Field

The present disclosure relates to a culture apparatus and a calibration method for a humidity sensor.

### Background Art

In a culture apparatus for incubating a culture such as a cell or a microorganism in a culture chamber, the culture chamber is maintained at a desired temperature (for example, 37°C) or a desired humidity (for example, 95% RH) by a heater or a vapor supply apparatus (for example, see Patent Literature (hereinafter, referred to as "PTL") 1).

### Citation List

### Patent Literature

PTL 1
Japanese Patent Application Laid-Open No. H5-227942

### Summary of Invention

### Technical Problem

The culture apparatus measures the humidity in the culture chamber by a humidity sensor and maintains the humidity at a desired humidity. The humidity sensor is subject to change with time, and in order to maintain the accuracy of the humidity sensor, regular calibration (adjustment) is essential. However, for the calibration, another measuring instrument for measuring the humidity is needed, and a measuring instrument that can measure a high humidity with high accuracy is very expensive. It is thus not easy for a user to perform the calibration or to manage the calibration.

An object of the present disclosure is to provide a culture apparatus and a calibration method for a humidity sensor that is capable of calibrating the humidity sensor without using another measuring instrument.

### Solution to Problem

A culture apparatus according to the present disclosure includes:
a first vapor supplier that humidifies a culture chamber;
a humidity sensor that detects a humidity in the culture chamber; and
a calibrator that brings the humidity in the culture chamber to a known equilibrium humidity by the first vapor supplier and calibrates the humidity sensor based on a measured value of the humidity sensor and the equilibrium humidity.

A calibration method according to the present disclosure is a calibration method for a humidity sensor that detects a humidity in a culture chamber, the calibration method including:
humidifying the culture chamber to bring the humidity in the culture chamber to a known equilibrium humidity;
detecting by the humidity sensor the humidity in the culture chamber brought to the equilibrium humidity; and
calibrating the humidity sensor based on a measured value of the humidity sensor and the equilibrium humidity.

### Advantageous Effects of Invention

According to the present disclosure, it is possible to calibrate the humidity sensor without using another measuring instrument.

### Brief Description of Drawings

FIG. 1 is a perspective view showing an example of a culture apparatus according to an embodiment of the present disclosure;
FIG. 2 is a front view showing an outline of a culture chamber of the culture apparatus shown in FIG. 1;
FIG. 3 is a schematic sectional view of the culture apparatus shown in FIG. 1 as seen from the right side; and
FIG. 4 is a flowchart for describing an example of a calibration method for a humidity sensor according to the embodiment of the present disclosure.

### Description of Embodiments

Hereinafter, embodiments of the present disclosure will be described in detail based on the drawings. The embodiment to be described below is an example, and the present disclosure is not limited thereto.

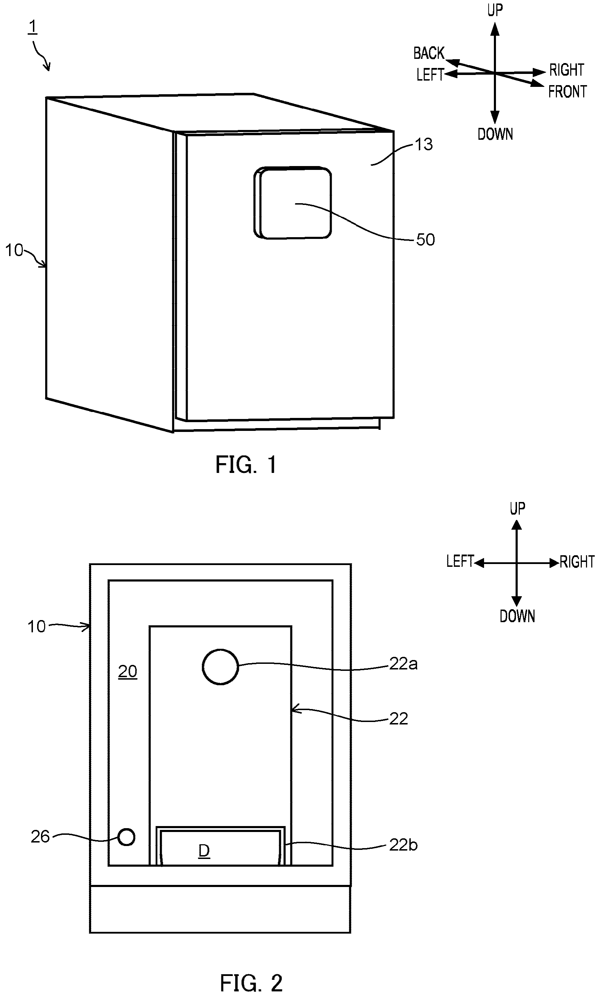

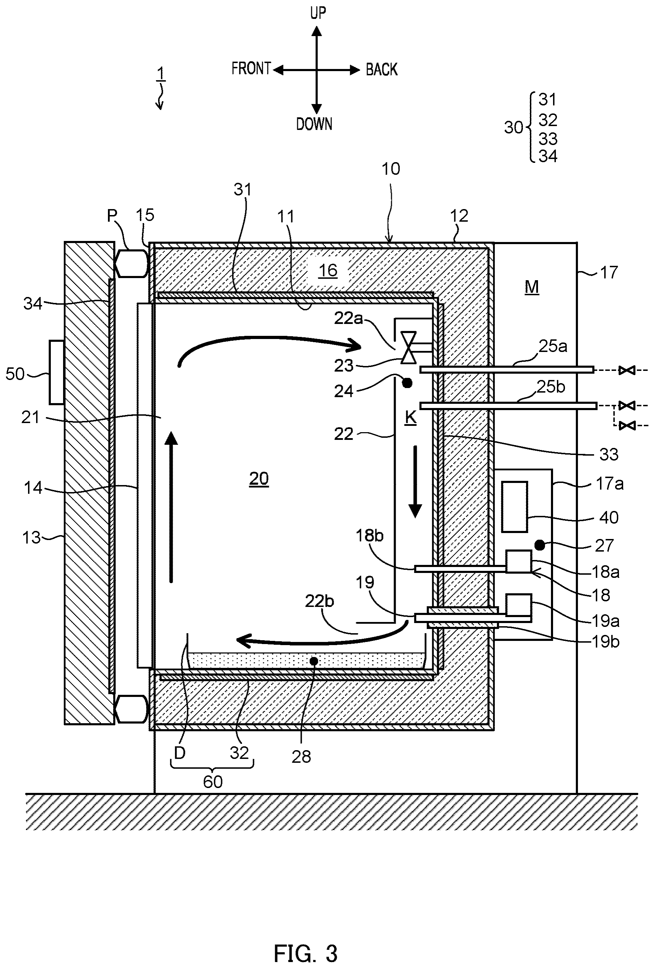

FIG. 1 is a perspective view showing an example of culture apparatus 1 according to the present embodiment. FIG. 2 is a front view showing an outline of culture chamber 20 of culture apparatus 1 shown in FIG. 1. FIG. 3 is a schematic sectional view of culture apparatus 1 shown in FIG. 1 as viewed from the right side.

In FIGS. 1 to 3, a side on which a user faces when the user uses culture apparatus 1 is referred to as a front side (front surface side) of culture apparatus 1, and an opposite side of the side on which the user faces is referred to as a back side (back surface side) of culture apparatus 1. In addition, the left side and the right side of the user when the user views culture apparatus 1 from the front are referred to as the left side and the right side of culture apparatus 1. Further, the side of culture apparatus 1, which is away from the surface on which culture apparatus 1 is installed, will be referred to as the upper side (top surface side) of culture apparatus 1, and the side opposite to the upper side will be referred to as the lower side (bottom surface side) of culture apparatus 1.

Culture apparatus 1 is an apparatus that cultivates a culture, such as a cell or a microorganism, in culture chamber 20 provided inside substantially box-shaped housing 10. The temperature, the humidity, the O₂ (oxygen) concentration, and the CO₂ (carbon dioxide) concentration in culture chamber 20 are held within appropriate ranges such that the atmosphere in culture chamber 20 is appropriate for the cultivation of the culture. As shown in FIG. 3, housing 10 includes inner box 11, outer box 12, outer door 13, inner door 14, and front plate 15.

Inner box 11 has a substantially box shape, includes culture chamber 20 on the inside thereof, and includes opening 21 of culture chamber 20 at the front surface thereof. Outer box 12 has a substantially box shape and covers a portion other than opening 21 on the outside of inner box 11. Inner box 11 and outer box 12 are made of a metal plate. Heat insulation material 16 is disposed between inner box 11 and outer box 12. Heat insulation material 16 is formed by, for example, bonding a plurality of plate-shaped insulating members to each other by an adhesive.

Outer door 13 and inner door 14 open and close opening 21. Packing P is disposed at the outer edge of outer door 13.

Front plate 15 is disposed on the front of inner box 11 and outer box 12 and connects inner box 11 to outer box 12 at the peripheral edge of opening 21. Front plate 15 is a plate member having a substantially rectangular outer periphery, and connects the front end of inner box 11 and the front end of outer box 12 over the entire periphery of opening 21.

Housing 10 includes heating unit 30 that heats culture chamber 20. Heating unit 30 includes a plurality of heaters, and each of the heaters is formed in a plate shape. Here, heating unit 30 includes heaters 31 to 34 and a side heater (not shown) as an example. Heaters 31 to 34 and the side heater are used in a normal operation mode, a dry heat sterilization operation mode, and a calibration mode to be described later, and are controlled such that the temperature distribution in culture chamber 20 is uniform.

Heaters 31 to 33 and the side heater are disposed outside inner box 11. Specifically, heater 31 is disposed on the top surface of inner box 11. Heater 32 is disposed on the bottom surface of inner box 11. Heater 33 is disposed on the back surface of inner box 11. The side heater is disposed on each of the right side surface and the left side surface of inner box 11. Heater 34 is disposed on a surface on the opening 21 side of outer door 13.

Heaters 31 to 34 and the side heater have, for example, a metal plate and a cord heater. The cord heater is disposed such that the temperature distribution of the metal plate is uniform. The cord heater may include one cord heater or may include a plurality of cord heaters connected in series.

Heating unit 30 described above is an example, and the number of heaters constituting heating unit 30, the arrangement of the heaters, the configuration of the heaters themselves, and the like can be appropriately changed. For example, the amount of heat released from opening 21 is larger than the amount of heat released from other parts, and thus a heater may be further provided around opening 21 to compensate for the amount of heat released from opening 21.

Further, duct 22 that vertically extends on the inner rear surface of inner box 11 is disposed in culture chamber 20. Gas passage K is formed inside duct 22. In this gas passage K, circulation fan 23 is disposed. By activating circulation fan 23, air in culture chamber 20 is sucked in through suction port 22a formed in an upper portion of duct 22, and this air is blown out to culture chamber 20 through blow-out port 22b provided in a lower portion of duct 22. Thus, forced circulation of the air as indicated with the bold arrows is performed. Within duct 22, temperature sensor 24 and gas supply apparatuses 25a and 25b are disposed.

Temperature sensor 24 detects the temperature in culture chamber 20. Specifically, temperature sensor 24 is disposed in the vicinity of suction port 22a and detects the temperature of air sucked in through suction port 22a.

Gas supply apparatuses 25a and 25b supply culture chamber 20 with an adjustment gas (for example, O₂ gas, N₂ (nitrogen) gas, and CO₂ gas) for adjusting the O₂ gas concentration and the CO₂ gas concentration in culture chamber 20.

Further, as illustrated in FIG. 2, Humidity sensor 26 is disposed on the inner rear surface of culture chamber 20. Humidity sensor 26 detects the humidity in culture chamber 20. In a lower portion of the inner rear surface of culture chamber 20, humidity sensor 26 is disposed on the left side of blow-out port 22b. The position of humidity sensor 26 shown in FIG. 2 is an example and can be appropriately changed.

Humidification tray D that stores a liquid (specifically water) that turns into vapor for humidification is installed between the lower portion of duct 22 and the bottom surface of inner box 11. Water stored in humidification tray D (hereinafter, referred to as "stored water") is sterilized by ultraviolet irradiation performed by a UV lamp (not illustrated).

The stored water in humidification tray D evaporates (natural vaporization) substantially in proportion to the difference between the saturated vapor pressure according to the temperature of the water and the vapor pressure of gas-phase water within culture chamber 20. As described above, humidification tray D constitutes a vapor supplier (the first vapor supplier in the present disclosure) that supplies vapor to culture chamber 20 through natural vaporization. In the case of this configuration, vapor is supplied to culture chamber 20 through natural vaporization, and thus, the amount of vapor to be supplied varies depending on the humidity in culture chamber 20. The humidity in culture chamber 20 eventually reaches an equilibrium state at a predetermined humidity.

The stored water of humidification tray D may be heated by heater 32 disposed on the bottom surface outside inner box 11 to change the temperature of the water. In this case, humidification tray D and heater 32 form vapor supplier 60 (first vapor supplier in the present disclosure) that supplies the vapor to culture chamber 20 by natural evaporation. In addition, the temperature of the stored water of humidification tray D is detected by water temperature sensor 28 to be described later. Here, heater 32 is referred to as heating that it warms the stored water of humidification tray D to a temperature below the boiling point. The stored water of humidification tray D is heated by heater 32 and is naturally evaporated. Even in a case where the above configuration is applied, since the vapor is supplied to culture chamber 20 by natural evaporation, the amount of vapor supplied changes according to the humidity in culture chamber 20, and the humidity in culture chamber 20 eventually reaches an equilibrium state at a predetermined humidity.

In the present embodiment, in a case where the humidity in culture chamber 20 that is the target value is relatively high, the humidification is performed using vapor supplier 60 having humidification tray D and heater 32. On the other hand, in a case where the target humidity level of culture chamber 20 is relatively low, the humidification may be performed without using heater 32 (without heating), by using a vapor supplier that includes only humidification tray D.

As shown in FIG. 3, the back surface and the bottom surface of outer box 12 of housing 10 are covered with cover 17. The space between the back surface of outer box 12 and cover 17 forms mechanical room M for disposing various apparatuses. Electrical box 17a is provided in mechanical room M. Control apparatus 40 and the like are housed in electrical box 17a.

Culture apparatus 1 further includes vapor supply apparatus 18, dehumidification member 19, outside air temperature sensor 27, and water temperature sensor 28. Outside air temperature sensor 27 detects a temperature around culture apparatus 1. Water temperature sensor 28 detects a temperature of the stored water of humidification tray D. The temperature of the stored water in humidification tray D may be estimated based on the output (for example, the amount of energization) of heater 32 that heats the stored water, and in this case, water temperature sensor 28 may not be installed.

Vapor supply apparatus 18 supplies vapor to culture chamber 20. Vapor supply apparatus 18 includes vapor generator 18a and vapor feeder 18b.

Vapor generator 18a is disposed in electrical box 17a, and includes a heater (not illustrated). Vapor generator 18a is supplied with water from a tank (not illustrated), in which water for vapor generation is stored, by a pump (not illustrated), and generates vapor by heating the water with the heater to evaporate the water. Vapor feeder 18b has a tubular shape, and supplies the vapor generated by vapor generator 18a to culture chamber 20.

In vapor supply apparatus 18, the water supplied from the tank is heated by the heater and is subjected to forced vaporization. Here, the water supplied from the tank is heated to a temperature equal to or higher than the boiling point thereof and is evaporated, which is therefore called heating. Vapor generator 18a and vapor feeder 18b of vapor supply apparatus 18 constitute a vapor supplier (the second vapor supplier in the present disclosure) that supplies vapor to culture chamber 20 through forced vaporization. Since vapor supply apparatus 18 supplies vapor to culture chamber 20 through forced vaporization, the amount of vapor to be supplied is independent of the humidity in culture chamber 20, and is capable of supplying a desired amount of vapor.

For example, when control apparatus 40 controls the pump, the amount per unit time of water to be supplied to vapor generator 18a is adjusted, and the amount per unit time of vapor to be supplied to culture chamber 20 is adjusted.

Dehumidification member 19 dehumidifies culture chamber 20 to prevent the condensation in culture chamber 20 and functions as a humidity regulator. Dehumidification member 19 is made of metal and has a rod shape. A first end of dehumidification member 19 is located above humidification tray D within culture chamber 20. A second end of dehumidification member 19 is located within electrical box 17a. Cooling apparatus 19a (for example, a Peltier element) for cooling dehumidification member 19 is attached to the second end of dehumidification member 19. Heat insulation material 19b is wound between the first end and second end of dehumidification member 19.

Control apparatus 40 controls cooling apparatus 19a such that the temperature of the first end portion of dehumidification member 19 is lower than the room temperature of culture chamber 20 based on the detection values of temperature sensor 24 and outside air temperature sensor 27 in culture chamber 20. Should the humidity in culture chamber 20 become relatively high, water droplets are generated only at the first end of dehumidification member 19. That is, it is possible to prevent the condensation from occurring on other parts (for example, the inner surface of inner box 11) and the culture in culture chamber 20.

The control of dehumidification member 19 may be performed based on the detection value of humidity sensor 26 in addition to temperature sensor 24 and outside air temperature sensor 27. In this case, when the detection value of humidity sensor 26 is equal to or higher than a predetermined threshold defined in advance, cooling apparatus 19a is controlled such that the temperature of the first end portion of dehumidification member 19 is lower than the room temperature of culture chamber 20. As a result, in a case where the detection value of humidity sensor 26 is lower than the predetermined threshold, the humidity in culture chamber 20 can be increased early. The predetermined threshold is a value lower than 100% RH, and is, for example, 90% RH.

Water droplets generated at the first end portion of dehumidification member 19 fall onto humidification tray D, and are sterilized by ultraviolet irradiation from the UV lamp. Therefore, even in a case where the water droplets are generated, the water droplets are prevented from having a negative effect on the culture.

Culture apparatus 1 receives an instruction to start and stop culture apparatus 1, a setting of the operation mode, and an input of various setting values of culture chamber 20 from operation section 50 disposed on outer door 13. The various setting values of culture chamber 20 are a set temperature, set humidity, a set concentration of O₂ gas, a set concentration of CO₂ gas, and the like. Control apparatus 40 controls circulation fan 23, gas supply apparatuses 25a, 25b, heating unit 30, and the like based on the input from operation section 50. Control apparatus 40 functions as a calibrator that calibrates humidity sensor 26 in the calibration mode to be described later. Operation section 50 includes a display that displays the status of culture apparatus 1.

Culture apparatus 1 has at least a normal operation mode (cultivation operation mode), a dry heat sterilization operation mode, and a calibration mode as its operation mode. The user selects the operation mode by operating operation section 50.

The normal operation mode is a mode in which circulation fan 23, gas supply apparatuses 25a, 25b, heating unit 30, vapor supply apparatus 18, dehumidification member 19, vapor supplier 60, and the like are operated such that the cultivation atmosphere, the cultivation humidity, and the cultivation temperature in culture chamber 20 are suitable for the cultivation of the culture. The cultivation atmosphere (concentration of O₂, N₂, and CO₂ gases), the cultivation humidity, and the cultivation temperature are set by the user inputting the values from operation section 50. In the normal operation mode, water is stored in humidification tray D, culture chamber 20 is humidified such that the humidity in culture chamber 20 becomes the cultivation humidity (for example, 95% RH), and the temperature in culture chamber 20 is maintained at the cultivation temperature (for example, 37°C).

The dry heat sterilization operation mode is a mode in which circulation fan 23, heating unit 30, and the like are operated to perform dry heat sterilization in culture chamber 20. In the dry heat sterilization operation mode, since the dry heat sterilization is performed, humidification tray D is empty, and the temperature in culture chamber 20 is maintained at the sterilization temperature (for example, 180°C).

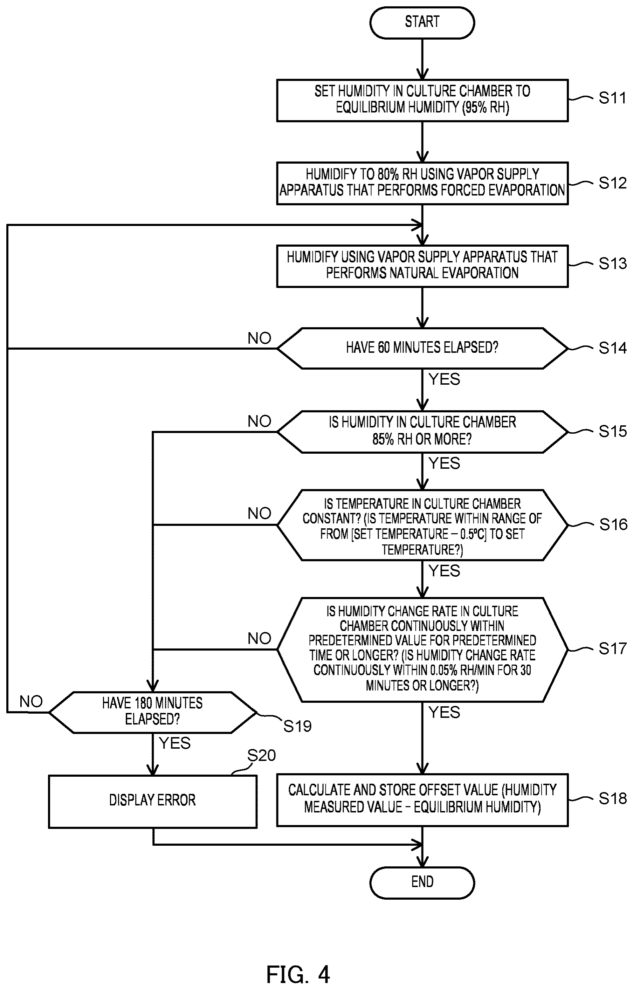

The calibration mode is a mode in which humidity sensor 26 is calibrated by operating vapor supply apparatus 18, vapor supplier 60, and the like such that culture chamber 20 is in a constant humidity environment. A calibration method for humidity sensor 26 in the calibration mode will be described below with reference to FIG. 4.

FIG. 4 is a flowchart for describing an example of a calibration method for humidity sensor 26 according to the present embodiment.

First, the user selects the calibration mode by operating operation section 50, and the following steps are performed. In the calibration mode, a sufficient amount of water is needed to bring the humidity in culture chamber 20 to the equilibrium humidity, so that, for example, the display of operation section 50 displays to request for installation of humidification tray D in which water is stored or the supply of water to humidification tray D.

### (Step S11)

Control apparatus 40 sets the humidity in culture chamber 20 to the equilibrium humidity (for example, 95% RH). The equilibrium humidity is predetermined, and when the user selects the calibration mode, the equilibrium humidity is automatically set. At this time, the set temperature in culture chamber 20 is also automatically set, and control apparatus 40 controls heating unit 30 such that the temperature becomes the set temperature.

Here, the equilibrium humidity will be described. In a case where the natural evaporation is performed, culture chamber 20 is in an equilibrium state at a humidity determined by the temperature in culture chamber 20 and the temperature of the stored water of humidification tray D, and is stabilized. In the present embodiment, the humidity in the equilibrium state is referred to as equilibrium humidity. In the present embodiment, the equilibrium humidity determined by the temperature in culture chamber 20 and the temperature of the stored water of humidification tray D is obtained in advance. For example, in the manufacturing process of culture apparatus 1, the above-described equilibrium temperature is obtained at the time of the operation check. In the calibration mode, when the known equilibrium humidity obtained in advance is set, control apparatus 40 controls heating unit 30 to control the temperature in culture chamber 20 and the temperature of the stored water of humidification tray D such that the equilibrium humidity is achieved.

In particular, in the present embodiment, the maximum equilibrium humidity that can be reached in culture chamber 20 is obtained in advance and used as the equilibrium humidity. The equilibrium humidity is determined by the temperature in culture chamber 20 and the temperature of the stored water of humidification tray D, and thus can be changed by the combination of the temperature in culture chamber 20 and the temperature of the stored water of humidification tray D, but according to the knowledge of the inventors and the like, the maximum equilibrium humidity is uniquely determined by the configuration of culture apparatus 1. Therefore, the maximum equilibrium humidity can be used as the most reliable reference humidity when humidity sensor 26 is calibrated.

As the configuration of culture apparatus 1 described above, at least the volume of culture chamber 20, the surface area of humidification tray D, and the output (for example, the amount of energization) of heater 32 disposed on the bottom surface of inner box 11 are determined, and thus the maximum equilibrium humidity is uniquely determined. In this case, basically, the maximum equilibrium humidity is uniquely determined by the humidification by vapor supplier 60 (the stored water of humidification tray D).

In addition, in a case where dehumidification member 19 is operated, the maximum equilibrium humidity in this case is uniquely determined when the output voltage for operating dehumidification member 19 is also determined. That is, in this case, basically, the maximum equilibrium humidity is uniquely determined by the humidification by vapor supplier 60 (the stored water of humidification tray D) and the dehumidification by dehumidification member 19.

Although the maximum equilibrium humidity is used as the equilibrium humidity here, as described above, a known equilibrium humidity other than the maximum equilibrium humidity obtained in advance may be used since the equilibrium humidity is determined by the temperature in culture chamber 20 and the temperature of the stored water of humidification tray D. In addition, although it takes time to calibrate, a plurality of known equilibrium humidities, including the maximum equilibrium humidity, may be used as the equilibrium humidity.

Hereinafter, the description will be made taking 95% RH that is the maximum equilibrium humidity as an example of the set equilibrium humidity.

### (Step S12)

Control apparatus 40 humidifies the inside of culture chamber 20 to 80% RH (rapid humidification) by using vapor supply apparatus 18 that performs the forced evaporation. At this time, control apparatus 40 refers to the humidity detected by humidity sensor 26 to humidity the inside of culture chamber 20 to 80% RH. 80% RH is a humidity that does not exceed the equilibrium humidity, and is a reference humidity for switching to vapor supplier 60 that performs the natural evaporation. The actual humidity may not be 80% RH as long as humidity sensor 26 before the calibration detects 80% RH. In addition, 80% RH is an example, and may be changed according to the set equilibrium humidity, for example.

In the present calibration mode, the humidification by vapor supply apparatus 18 is not always needed. For example, when the humidity in culture chamber 20 when the calibration mode is started is a high humidity, such as 80% RH or more, present step S12 may be skipped. On the other hand, in a case where the humidity in culture chamber 20 when the calibration mode is started is not the high humidity, for example, in a case where the humidity is less than 80% RH, present step S12 is better executed to reduce the time needed for the calibration mode.

### (Step S13)

Control apparatus 40 humidifies the inside of culture chamber 20 by using vapor supplier 60 that performs the natural evaporation. As described above, in a case where the natural evaporation is performed, culture chamber 20 is brought to the equilibrium state at an equilibrium humidity determined by the temperature in culture chamber 20 and the temperature of the stored water of humidification tray D, and is stabilized.

### (Step S14)

Control apparatus 40 confirms whether 60 minutes have elapsed after the calibration mode is started. When 60 minutes have elapsed (YES), the process proceeds to step S15, and when 60 minutes have not elapsed (NO), the process returns to step S13. 60 minutes is an example, and is the time for the humidity in culture chamber 20 to become 85% RH or more in a case where vapor supply apparatus 18 and vapor supplier 60 operate without any problem, in the present embodiment. That is, the time in the present step may be set to the time taken for the humidity in culture chamber 20 to reach a high humidity (for example, 85% RH) and may be changed according to the set equilibrium humidity.

### (Step S15)

Control apparatus 40 uses humidity sensor 26 to confirm whether the humidity in culture chamber 20 is 85% RH or more. When the humidity is 85% RH or more (YES), the process proceeds to step S16, and when the humidity is not 85% RH or more (NO), the process proceeds to step S19. Also at this step, control apparatus 40 uses humidity sensor 26 to confirm whether the humidity in culture chamber 20 is 85% RH or more. 85% RH is a reference humidity for confirming whether the humidity in culture chamber 20 is high. The actual humidity may not be 85% RH as long as humidity sensor 26 before the calibration detects 85% RH. In addition, 85% RH is an example, and may be changed according to the set equilibrium humidity, for example.

In present step S15, control apparatus 40 monitors the humidity in culture chamber 20 and confirms whether culture chamber 20 is in a high humidity state by considering a case exemplified below or the like.

For example, in a case where the user forgets to put water in humidification tray D, culture chamber 20 is not in a high humidity state. Therefore, control apparatus 40 is configured to repeat the humidification in step S13 and the confirmation of the humidity in present step S15 for 180 minutes specified in step S19 described below. Then, control apparatus 40 proceeds to following step S20 to display the error when culture chamber 20 is not in a high humidity state even when 180 minutes have elapsed.

In addition, immediately after culture apparatus 1 is turned on, the humidity in culture chamber 20 may be low, and in this case, it takes time to increase the humidity in culture chamber 20 to a high humidity. Therefore, control apparatus 40 is configured to repeat the humidification in step S13 and the confirmation of the humidity in present step S15 for 180 minutes specified in step S19. Then, when the humidity in culture chamber 20 is increased to a high humidity within 180 minutes, control apparatus 40 is configured to proceed to step S16.

As described above, in present step S15, control apparatus 40 confirms whether culture chamber 20 is in a high humidity state, and does not proceed to following step S18 when culture chamber 20 is not in a high humidity state, thereby preventing humidity sensor 26 from being erroneously calibrated.

### (Step S16)

Control apparatus 40 uses temperature sensor 24 to determine whether the temperature in culture chamber 20 is constant. When the temperature can be determined to be constant (YES), the process proceeds to step S17, and when the temperature cannot be determined to be constant (NO), the process proceeds to step S19. Control apparatus 40 determines that the temperature detected by temperature sensor 24 is constant when the temperature is within a range of, for example, [set temperature - 0.5°C] to set temperature. As an example, when the set temperature is 50°C, the temperature is determined to be constant when the temperature is in a range of from 49.5°C to 50°C.

In present step S16, control apparatus 40 monitors the temperature in culture chamber 20 and confirms whether the temperature in culture chamber 20 is constant at the set temperature by considering a case exemplified below or the like.

For example, immediately after culture apparatus 1 is turned on, the temperature in culture chamber 20 is low. Therefore, for 180 minutes specified in step S19, control apparatus 40 controls heating unit 30 such that the temperature in culture chamber 20 is the set temperature, and repeats the confirmation of the temperature in present step S16. Then, when the temperature in culture chamber 20 becomes constant at the set temperature within 180 minutes, control apparatus 40 is configured to proceed to step S17.

As described above, in present step S16, control apparatus 40 confirms whether the temperature in culture chamber 20 is constant at the set temperature, and does not proceed to following step S18 when the temperature is not constant at the set temperature, thereby preventing humidity sensor 26 from being erroneously calibrated. For example, in a case where the temperature in culture chamber 20 is lower than the set temperature, the relative humidity is increased, but in present step S16, the process does not proceed to step S18 when the temperature in culture chamber 20 is lower than the set temperature, so that humidity sensor 26 is not erroneously calibrated.

### (Step S17)

Control apparatus 40 uses humidity sensor 26 to determine whether the humidity change rate in culture chamber 20 is continuously within a predetermined value for a predetermined time or longer. When the humidity change rate is continuously within the predetermined value for the predetermined time or longer (YES), the process proceeds to step S18, and when the humidity change rate is not continuously within the predetermined value for the predetermined time or longer (NO), the process proceeds to step S19. Control apparatus 40 determines that the humidity change rate is continuously within a predetermined value for a predetermined time or longer when the humidity change rate is, for example, continuously within 0.05% RH/min for 30 minutes or longer. Even before the calibration of humidity sensor 26, the measurement accuracy of the humidity change is not affected, and thus the determination is made whether the humidity in culture chamber 20 is in the equilibrium state by determining whether the humidity change rate is continuously within a predetermined value for a predetermined time or longer.

In present step S17, control apparatus 40 monitors the humidity change rate in culture chamber 20 to confirm whether the humidity in culture chamber 20 is in the equilibrium state, that is, whether the humidity is in the equilibrium humidity. Even before the calibration of humidity sensor 26, the determination can be made whether the humidity in culture chamber 20 is the equilibrium humidity by monitoring the humidity change rate, and thus the calibration error in step S18 can be suppressed.

### (Step S18)

Control apparatus 40 uses humidity sensor 26 to detect the humidity in culture chamber 20 and obtain the humidity measured value, calculates a difference (humidity measured value - equilibrium humidity) between the humidity measured value and the equilibrium humidity as an offset value, and stores the offset value. Control apparatus 40 calibrates humidity sensor 26 using the stored offset value and controls the humidity in the normal operation mode, for example. As described above, control apparatus 40 can calibrate humidity sensor 26 based on the humidity measured value of humidity sensor 26 and the equilibrium temperature.

### (Step S19)

Control apparatus 40 confirms whether 180 minutes have elapsed after the calibration mode is started. When 180 minutes have elapsed (YES), the process proceeds to step S20, and when 180 minutes have not elapsed (NO), the process returns to step S13. 180 minutes is an example, and is the time for the humidity in culture chamber 20 to become 95% RH that is the equilibrium humidity in a case where vapor supply apparatus 18 and vapor supplier 60 operate without any problem, in the present embodiment. That is, the time in the present step may be set to the time for the equilibrium humidity (for example, 95% RH) in culture chamber 20 to be achieved.

### (Step S20)

Control apparatus 40 displays the error on the display of operation section 50. As an example, as described above, when culture chamber 20 is not in a high humidity state, the error indicating this condition is displayed.

As described above, in the present embodiment, the calibration of humidity sensor 26 is performed using the known equilibrium humidity obtained in advance in culture apparatus 1, and thus the user can easily calibrate humidity sensor 26 without using another expensive measuring instrument. A simple calibration method for calibrating humidity sensor 26 is provided for the user.

In the present embodiment, in a case where the maximum equilibrium humidity described above is used as the equilibrium humidity to calibrate humidity sensor 26, the calibration error can be further suppressed since the maximum equilibrium humidity is uniquely determined as described above.

In the present embodiment, in a case where the operation condition (for example, the temperature and the humidity) in the normal operation mode and the operation condition (for example, the temperature and the humidity) in the calibration mode are substantially the same, the calibration of humidity sensor 26 can be performed while the culture is being continuously performed (with equipment installed in the chamber left in place).

In the present embodiment, when the operation mode is changed from the normal operation mode to the calibration mode, the time needed to bring the humidity in culture chamber 20 to the equilibrium humidity can be shortened when the operation condition in the normal operation mode (for example, the temperature and the humidity) and the operation condition in the calibration mode (for example, the temperature and the humidity) are close to each other. As a result, it is possible to shorten the time needed to calibrate humidity sensor 26.

In the present embodiment, since culture apparatus 1 has dehumidification member 19, dehumidification member 19 can be used to calibrate humidity sensor 26 while preventing the condensation in culture chamber 20.

In the present embodiment, humidity sensor 26 has a temperature detector that detects a temperature around the sensor and a humidity detector that detects a humidity around the sensor. The amount of water vapor in culture chamber 20 may be calculated from the temperature and the humidity around the sensor, and the humidity in culture chamber 20 may be detected from the temperature of culture chamber 20. As a result, the humidity in culture chamber 20 can be accurately detected regardless of the temperature distribution in culture chamber 20, that is, regardless of the position of humidity sensor 26.

Thus, the embodiments of the present disclosure have been described. It should be understood, however, that the above description is merely illustrative of preferred embodiments of the present disclosure and is not intended to limit the scope thereof. That is, the descriptions of the structures of the above apparatus and the shapes of respective components are merely examples, and it is apparent that various modifications and additions may be made within the scope of the present disclosure.

The disclosure of Japanese Patent Application No. 2023-085709, filed on May 24, 2023, including the specification, drawings and abstract, is incorporated herein by reference in its entirety.

### Industrial Applicability

The present disclosure is useful for a culture apparatus and a calibration method for a humidity sensor.

### Reference Signs List

1 Culture apparatus
10 Housing
11 Inner box
12 Outer box
13 Outer door
14 Inner door
15 Front plate
16 Heat insulation material
17 Cover
18 Vapor supply apparatus
19 Dehumidification member
20 Culture chamber
21 Opening
22 Duct
23 Circulation fan
24 Temperature sensor
25a, 25b Gas supply apparatus
26 Humidity sensor
27 Outside air temperature sensor
28 Water temperature sensor
30 Heating unit
31, 32, 33, 34 Heater
40 Control apparatus
50 Operation section
60 Vapor supplier

## Claims

1. A culture apparatus, comprising:
a first vapor supplier that humidifies a culture chamber;
a humidity sensor that detects a humidity in the culture chamber; and
a calibrator that brings the humidity in the culture chamber to a known equilibrium humidity by the first vapor supplier and calibrates the humidity sensor based on a measured value of the humidity sensor and the equilibrium humidity.

2. The culture apparatus according to claim 1, wherein
the first vapor supplier supplies vapor to the culture chamber by natural evaporation.

3. The culture apparatus according to claim 1, wherein
the calibrator calculates a difference between the measured value and the equilibrium humidity as an offset value and calibrates the humidity sensor using the offset value.

4. The culture apparatus according to claim 1, wherein
in a case where a change rate of the measured value is continuously within a predetermined value for a predetermined time or longer, the calibrator determines that the humidity in the culture chamber is the equilibrium humidity.

5. The culture apparatus according to claim 4, further comprising:
a temperature sensor that detects a temperature in the culture chamber, wherein
in a case where the temperature in the culture chamber is constant, the calibrator performs a determination as to whether the humidity in the culture chamber is the equilibrium humidity.

6. The culture apparatus according to claim 2, further comprising:
a second vapor supplier that supplies vapor into the culture chamber by forced evaporation, wherein
the calibrator supplies the vapor by the second vapor supplier to a humidity that does not exceed the equilibrium humidity.

7. The culture apparatus according to claim 2, further comprising:
a humidity regulator that prevents condensation in the culture chamber, wherein
the calibrator brings the humidity in the culture chamber to the equilibrium humidity by the first vapor supplier and the humidity regulator.

8. The culture apparatus according to claim 1, wherein
the equilibrium humidity is a maximum equilibrium humidity reachable in the culture chamber.

9. A calibration method for a humidity sensor that detects a humidity in a culture chamber, the calibration method comprising:
humidifying the culture chamber to bring the humidity in the culture chamber to a known equilibrium humidity;
detecting by the humidity sensor the humidity in the culture chamber brought to the equilibrium humidity; and
calibrating the humidity sensor based on a measured value of the humidity sensor and the equilibrium humidity.
